(19) Europäisches Patentamt
European Patent Office
Office européen des brevets

(11) EP 0 500 594 B1

(12) **EUROPEAN PATENT SPECIFICATION**

(45) Date of publication and mention
of the grant of the patent:
**14.01.1998 Bulletin 1998/03**

(51) Int. Cl.$^6$: **A61K 9/06**, A61K 9/16,
A61K 9/51

(21) Application number: **90915978.2**

(22) Date of filing: **01.11.1990**

(86) International application number:
**PCT/GB90/01676**

(87) International publication number:
**WO 91/06282 (16.05.1991 Gazette 1991/11)**

(54) **SMALL PARTICLE DRUG COMPOSITIONS**

KLEINE PARTIKELN ENTHALTENDE ARZNEISTOFFZUSAMMENSETZUNG

COMPOSITIONS PHARMACOLOGIQUES A PARTICULES DE TAILLE REDUITE

(84) Designated Contracting States:
**AT BE CH DE DK ES FR GB GR IT LI LU NL SE**

(30) Priority: **04.11.1989 GB 8924935**

(43) Date of publication of application:
**02.09.1992 Bulletin 1992/36**

(73) Proprietor:
**DANBIOSYST UK LIMITED**
**Nottingham NG7 2RQ (GB)**

(72) Inventor:
**ILLUM, Lisbeth**
**19 Cavendish Crescent North**
**Nottingham NG7 1BA (GB)**

(74) Representative:
**Bassett, Richard Simon**
**ERIC POTTER & CLARKSON**
**St. Mary's Court**
**St. Mary's Gate**
**Nottingham NG1 1LE (GB)**

(56) References cited:
EP-A- 0 122 036     WO-A-87/03197
WO-A-88/09163     WO-A-89/03207
DE-A- 3 341 001     FR-A- 2 371 454

- **Nato Asi Ser., Ser. A, volume 125, 1986, (Delivery Syst. Pept. Drugs), L. Illum: "Microspheres as a potential controlled release nasal drug delivery system", pages 205-210**
- **International Journal of Pharmaceutics, volume 39, no. 3, 1987, Elsevier Science Publishers B.V., L. Illum et al.: "Bioadhesive microspheres as a potential nasal drug delivery system", pages 189-199**
- **ACM Symp. Ser., Controlled release technol. pharm. Appl.), volume 348, 1987, S. S. Davis et al.: "Microspheres as controlled-release systems for parenteral and nasal administration", pages 201-213**

Remarks:
The file contains technical information submitted after the application was filed and not included in this specification

## Description

The present invention relates to drug compositions and more particularly to a small particle drug composition which provides for the uptake of active drug across the nasal mucosa.

There is a need to provide effective absorption of high molecular weight material such as proteins and peptides across biological membranes. Normally such molecules are not taken up by the body if administered to the gastrointestinal tract, the buccal mucosa, the rectal mucosa or the vaginal mucosa or if given as an intra-nasal system. Because peptide hormones such as insulin and calcitonin have a high molecular weight and are readily decomposed by proteolytic enzymes such as pepsin, aminopeptidases, trypsin and chymotrypsin, not enough is absorbed to display an effective pharmacological effect and accordingly they have been administered by parenteral injection.

However, since the administration by injection causes pain, various attempts have been made to develop alternative methods of administration.

Recent studies with insulin have demonstrated that the absorption of such a compound can be increased if it is given together with a so-called absorption enhancer, such as non-ionic surfactants and various bile salt derivatives. An increased permeability of membranes in the presence of these types of surfactant material is not unexpected, indeed the literature in the field of gastroenterology contains a wide range of such absorption promoters. (For a review see Davis *et al* (editors), Delivery Systems for Peptide Drugs. Plenum Press, New York 1987.) However, such materials will probably not be acceptable for the chronic administration of pharmacological agents because of their irritant effects on membranes. This includes not only the non-ionic variety of surface active agents but also bile salts and bile salt derivatives (e.g. fusidic acid).

At the present time the nose is being proposed as an alternative route for the delivery of drugs that will act within the systemic circulation. Particular attention is being focused on nature-identical peptides or proteins, or analogues or fragments thereof, produced by recombinant DNA techniques. Other drugs that are being suggested are those that are poorly absorbed orally or are extensively metabolised either in the gastrointestinal tract itself or are subject to first pass metabolism in the liver.

However, most polypeptide drugs show a low bioavailability when administered intranasally.

The rapid clearance of nasal sprays from the nose can probably be considered to be a major factor in influencing loss of drugs from potential absorption surfaces. In addition, in the case of peptides and proteins, enzymatic degradation of the drug and molecular size may also have a role in giving low bioavailabilities.

Our earlier co-pending application WO88/09163 discloses intra-nasal microsphere formulations containing an enhancer and our earlier co-pending application WO89/03207 discloses intra-nasal microsphere formulations containing drugs of molecular weight below 6000 which do not require an enhancer. In both of these applications, the diameter of the microspheres is in the range 10 μm to 100 μm. EP 122 036 (Teijin Ltd.) discloses powdery formulations for nasal administration in which at least 90 wt % of the particles have an effective diameter ranging from 10 μm to 250 μm.

It is taught in the art that particles for nasal delivery should be of diameter greater than 10 μm. EP 122 036 states that in compositions in which more than 10 wt % of the particles are below 10 μm, more particles will go further into the lungs or escape from the nostrils. It is known to use particles of diameter less than 10 μm for delivery of drugs to the lungs. GB 1 381 872 and GB 1 520 248 (Fisons) describe powdery compositions of particles less than 10 μm which are administered by oral inhalation to the lungs.

WO 87/03197 discloses a formulation comprising ion-exchange microspheres and incorporating sodium cromoglycate. The microspheres are preferably in the size range 1-200 μm and may have a diameter of 1-10 μm for administration as a pressurised aerosol to the nose. The sodium cromoglycate is locally acting at the site of administration.

DE 3341001 discloses nano-particles of 1 micron or less, which are made from synthetic polymers.

FR 2 371 454 discloses liquid or powder formulations comprising an ergot peptide alkaloid as the active agent. When the formulation is a powder, the particles are in the size range 20-32 μm.

It has now been found, surprisingly, that bio-adhesive microspheres of diameter less than 10 μm can be used effectively and advantageously to deliver drugs to the nasal mucosa.

The invention therefore provides a drug delivery composition for intranasal delivery comprising a plurality of bioadhesive microspheres and active drug associated with or forming at least part of each microsphere, at least 90 wt % of the microspheres having a diameter of 0.1 μm or more but less than 10 μm characterised in that the drug is one for which systemic delivery is desired.

The term "bioadhesive" as used herein is defined as a substance which adheres to the nasal mucosa, preferably to a greater extent than microcrystalline cellulose. It is thought that such bioadhesive microspheres interact with the glycoproteins in the mucus and/or the epithelial cells. The term "drug" is used to embrace any pharmacologically active agent, including hormones, polypeptides and vaccines or components thereof, for example isolated antigens or antigenic parts or mimics thereof.

For any particulate system consisting of a distribution of particle sizes, it is important to define exactly the way in which the diameter is measured. A powder system produced by milling or emulsification followed by suitable processing to yield microspheres (this includes both powders and bioadhesive microspheres) is expected to follow a so-called log normal distribution. Particle size measured by microscopic observation will give a number average distribution. This can be converted to a weight distribution (number-weight, mean diameter), using equations found in standard text books such as T. Allen, *Particle Size Measurement*, second edition, Chapman and Hall, 1974 and Caserett, L. J. in *Toxicology*, edited by Casarett, L. J. and Doull, J., Macmillan, New York, 1975, chapter 9.

In the latter, it is stated that the customary expression of particle size is in terms of the median size, either count or mass. For a log normally distributed powder, conversion between a count median diameter (CMD) and a mass median diameter MMD is easily accomplished by a simple calculation where $\delta g$ is the geometric standard deviation:-

$$\log M \text{ (Count)} = \log M' \text{ (Mass)} - 6.9 \log^2 \delta g$$

The weight distribution can be measured directly by screening or sieving or by sedimentation balance. Details are given in the book by Allen (see above).

For a spherical particle, size is uniquely defined and it is possible to talk about a mean diameter. However, with non-spherical particles it is necessary to consider an effective diameter as the size of a sphere that corresponds to the particle under the chosen conditions of measurement. The various options are discussed in the book by T. Allen, where derived diameters are determined by measuring a size dependent property of the particle and relating it to a linear dimension. Effective diameter has been defined by Teijin, so far as it applies to their nasal delivery system, in EP 23359. They refer to a diameter as determined by the opening sizes of sieves. For example, a powder having an effective particle diameter (d) of $37 < d \leq 44$ passes through a sieve having an opening size of 44 $\mu m$ (microns) but does not pass through a sieve having an opening size of 37 $\mu m$ (microns).

A vibratory sieve may be used when the effective particle diameter of a powder is more than 37 $\mu m$ (microns), and a sonic sieve (Micro Hand Sifter SWM-2, a product of Tsutsui Rikagaku Kikai Co. Ltd.) may be used when the effective particle diameter of a powder is not more than 37 $\mu m$ (microns). It is believed that this definition also applies to EP 122 036 (Teijin Ltd.).

Thus, 90 wt % by weight of spherical microspheres of the present invention have a true mean weight diameter of less than the 10$\mu m$ effective diameter of the Teijin particles. Preferably 90 wt% of the microspheres are over 0.5 $\mu m$ in diameter, more preferably over 1.0$\mu m$ and most preferably over 2 $\mu m$ in diameter. Suitably, 95 wt % or 99 wt % of the particles satisfy one or more of these criteria.

Preferably the microspheres are prepared from a bio-compatible material that will gel in contact with the mucosal surface. Substantially uniform solid microspheres are preferred. Starch microspheres (crosslinked if necessary) are a preferred material. Other materials that can be used to form microspheres include starch derivatives, gelatin, albumin, collagen, dextran and dextran derivatives. Preparation of these microspheres is well described in the pharmaceutical literature (see for example Davis *et al.*, (Eds), "*Microspheres and Drug Therapy*", Elsevier Biomedical Press, 1984, which is incorporated herein by reference). Emulsion and phase separation methods are both suitable. For example, albumin microspheres may be made using the water-in-oil emulsification method where a dispersion of albumin is produced in a suitable oil by homogenization techniques or stirring techniques, with the addition if necessary of small amounts of an appropriate surface active agent. The size of the microspheres is largely dictated by the speed of stirring or homogenization conditions. The agitation can be provided by a simple laboratory stirrer or by more sophisticated devices such as a microfluidizer or homogenizer. Emulsification techniques are also used to produce starch microspheres as described in GB 1 518 121 and EP 223 303 as well as for the preparation of microspheres of gelatin. Proteinaceous microspheres may also be prepared by coacervation methods such as simple or complex coacervation or by phase separation techniques using an appropriate solvent or electrolyte solution. Full details of the methods of preparing these systems can be obtained from standard text books (see for example Florence and Attwood, *Physicochemical Principles of Pharmacy* 2nd Ed., MacMillan Press, 1988, Chapter 8).

The microspheres obtained may be sieved if necessary in order to separate out microspheres in the desired size range. Other size separation techniques (air elutriation) may also be employed. The final microspheres can be modified by chemical cross-linking or heat treatment. The active agent can be incorporated into the microspheres during their formulation or sorbed into/onto the system after preparation. The effectiveness of the system can be controlled by the physical nature of the microsphere matrix and, for example, the extent of cross linking. The microsphere delivery systems may also include microspheres made from the active peptide or protein itself such as insulin microspheres, using methods analogous to those used to form albumin microspheres.

As an added advantage the particles may have variable controlled release characteristics through modifications made to the microsphere system, for example by controlling the degree of cross-linking or by the incorporation of excipients that alter the diffusional properties of the administered drug. The amount of drug that can be carried by the microspheres is termed the loading capacity, which is determined by the physicochemical properties of the drug molecule and in particular its size and affinity for the particle matrix.

Higher loading capacities are to be expected when the administered drug is incorporated into the microspheres during the actual process of microsphere manufacture. It is known that for many peptides and proteins the amount of drug substance to be administered for a resultant therapeutic effect will be of the order of a few micrograms or less. Microcapsules of a similar size, which are bioadhesive and which have controlled release properties, may also provide similar benefit in terms of an increased and modified bio-availability of administered drugs. These microcapsules can be produced by a variety of methods. The surface of the capsule can be adhesive in its own right or can be modified by coating methods familiar to those skilled in the art. These coating materials are preferably bioadhesive polymers such as polycarbophil, carbopol, DEAE-dextran or alginates. These microcapsules are deemed to be "microspheres" for the purposes of this specification and, again, are more than 0.1 $\mu$m in diameter but less than 10 $\mu$m.

Using the combination of microspheres and drug, it has been found that the bioadhesive microsphere systems have the ability to enhance greatly the bioavailability of drugs, especially polar drugs, when they are administered together.

This potentiation of effect is believed to be due to the greater retention of the delivery systems in the nasal cavity.

The microsphere composition can also afford protection of the drug against degradation by enzymes.

The drug delivery system of the invention may advantageously comprise an absorption enhancer. By "enhancer", we mean any material which acts to increase absorption across the mucosa. Such materials include mucolytic agents, degradative enzyme inhibitors and compounds which increase permeability of the mucosal cell membranes. Whether a given compound is an "enhancer" can be determined by comparing two formulations comprising a non-associated, small polar molecule as the drug, with or without the enhancer, in an in vivo or good model test and determining whether the uptake of the drug is enhanced to a clinically significant degree. The enhancer should not produce any problems in terms of chronic toxicity because in vivo the enhancer should be non-irritant and/or rapidly metabolised to a normal cell constituent that does not have any significant irritant effect.

A preferred enhancing material is the material lysophosphatidylcholine obtainable from egg or soy lecithin. Other lysophosphatidylcholines that have different acyl groups as well as lyso compounds produced from phosphatidylethanolamines and phosphatidic acid which have similar membrane modifying properties may be used. Acyl carnitines (e.g. palmitoyl-dl-carnitine-chloride) is an alternative. A suitable concentration is from 0.02 to 10%.

Other enhancing agents that are appropriate include chelating agents (EGTA, EDTA, alginates), surface active agents (especially non-ionic materials), acyl glycerols, fatty acids and salts, tyloxapol and biological detergents listed in the SIGMA Catalog, 1988, page 316-321. Also agents that modify the membrane fluidity and permeability are appropriate such as enamines (e.g. phenylalanine enamine of ethyl-acetoacetate), malonates (e.g. diethyleneoxymethylene malonate), salicylates, bile salts and analogues and fusidates. Suitable concentrations are up to 10%.

The same concept of delivery of a drug incorporated into or onto a bioadhesive microsphere with an added pharmaceutical adjuvant applies to systems that contain active drug and mucolytic agent, peptidase inhibitors or non-drug polypeptide substrate singly or in combination. Suitably mucolytic agents are thiol-containing compounds such as N-acetylcysteine and derivatives thereof. Peptide inhibitors include actinonin, amastatin, bestatin, chloroacetyl-HOLeu-Ala-Gly-NH$_2$, diprotin A and B, ebelactone A and B, E-64, leupeptin, pepstatin A, phisphoramidon, H-Thr-(tBu)-Phe-Pro-OH, aprotinin, kallikrein, chymostatin, benzamidine, chymotrypsin and trypsin. Suitable concentrations are from 0.01 to 5%. The person skilled in the art will readily be able to determine whether an enhancer should be included.

The microsphere composition may be used with drugs selected from the following list: insulin, calcitonins (for example porcine, human, salmon, chicken, or eel) and synthetic modifications thereof*, enkephalins*, LHRH and analogues* (Nafarelin, Buserelin, Zolidex), GHRH (growth hormone releasing hormone)*, nifedipin, THF(thymic humoral factor)*, CGRP (calcitonin gene related peptide)*, atrial natriuretic peptide*, antibiotics, metoclopramide*, ergotamine*, Pizotizin*, nasal vaccines (particularly HIV vaccines, measles, rhinovirus Type 13 and respiratory syncitial virus)*, pentamidine, CCK* (Cholecystikinine), DDVAP* and Interferons.

The starred drugs are especially preferred for administration with the microsphere system of the invention.

Further drugs include: antibiotics and antimicrobial agents such as tetracyline hydrochloride, leucomycin, penicillin, penicillin derivatives, erythromycin, sulphathiazole and nitrofurazone; local anaesthetics such as benzocaine; vasoconstrictors such as phenylephrine hydrochloride, tetrahydrozoline hydrochloride, naphazoline nitrate, oxymetazoline hydrochloride and tramazoline hydrochloride; cardiotonics such as digitalis and digoxin; vasodilators such as nitroglycerine and papaverine hydrochloride; antiseptics such as chlorhexidine hydrochloride, hexylresorcinol, dequaliniumchloride and ethacridine; enzymes such as lysozyme chloride, dextranase; bone metabolism controlling agents such as vitamin D, active vitamin D$_3$ and vitamin C; sex hormones; hypotensives; sedatives; anti-tumour agents; steroidal anti-inflammatory agents such as hydrocortisone, prednisone, fluticasone, prednisolone, triamcinolone, triamcinolone acetonide, dexamethasone, betamethasone, beclomethasone, and beclomethasone dipropionate; non-steroidal anti-inflammatory agents such as acetaminophen, aspirin, aminopyrine, phenylbutazone, medanamic acid, ibuprofen, diclofenac sodium, indomethacine, colchicine, and probenocid; enzymatic anti-inflammatory agents such as chymotrypsin and bromelain seratiopeptidase; anti-histaminic agents such as diphenhydramine hydrochloride, chloropheniramine maleate and clemastine; anti-allergic agents and antitussive-expectorant antasthmatic agents such as sodium chromoglycate, codeine phosphate, and isoproterenol hydrochloride.

The molecular weight of the drug is preferably in the range 100 to 300,000.

In order to improve the properties, appearance or odour of the pharmaceutical composition, it may, if desired, contain any of known additives such as colouring agents, preservatives or antiseptics. Examples of colouring agents include β-carotene, Red No. 2 and Blue No. 1; examples of preservatives include stearic acid, ascorbyl stearate and ascorbic acid; examples of antiseptics include p-hydroxy-benzoate, phenol and chlorobutanol; and examples of corrigents include menthol and citrus perfume.

A further embodiment of the invention provides a kit comprising a drug delivery composition and means to deliver the composition to the nasal mucosa in a gas stream. The gas stream may be air or any other physiologically harmless gas. Preferably the means is such that, in use, the product of the flow rate and the square of the microsphere diameter is greater than 2000 $\mu m^2$.litres/min.

The deposition in the nose will depend on two factors: the size of the particles (aerodynamic diameter $d_a$) and flow rate (F) of inspiratory air.

The controlling factor is $(d_a)^2 F$ where da is measured in microns and F in litres/min.

The product $(d_a)^2 F$ should exceed 2000$\mu m^2$.litres/min to give the required deposition in the nasal cavity of the total dose. Resting ventilation is of the order of 30 litres/min.

Under extreme exertion or rapid inhalation, a very large fraction of the deposition takes place within the anterior non-ciliated part of the nose, where particles are retained for long periods, gradually being dragged along to the nasopharynx by the mucus drag effect. Details of deposition and flow rate studies may be found in the art, for example G.M. Hidy, *Aerosols*, Academic Press Inc. 1984.

For particulate systems administered to the respiratory tract, it is necessary to consider the aerodynamic diameter that takes into account the size of the particle and its density. For example, a particle with a physical diameter of 0.5 μm and density of 10 will behave like a larger particle (of greater than 2 μm (microns)) of unit density. This applies strictly to spherical particles and may be varied markedly by the shape of the particle. The aerodynamic (kinetic diameter) has been defined as the diameter of a hypothetical sphere of unit density having the same terminal settling velocity as a particle in question regardless of its geometric size, shape and true density.

The microspheres, in for example a freeze dried form, can be administered to the nasal cavity using a nasal insufflator device or pressurised aerosol cannister. Examples of these are already employed for commercial powder systems intended for nasal application. The microspheres should be administered in a dry, air-dispersable form.

The small microspheres of the present invention have been found to be easier to administer using available devices, especially those working on the basis of pressure packs and accurate valves and actuators, as fewer problems with blockages occur.

Small microspheres are also easier to fluidize in powder administration devices, such as insufflators.

The narrower size range has been found to give a more uniform dose for an active material such as a peptide. The narrower size range has also been found to minimize separation of large and small particles on storage and transport and during administration. The admixture of insulin and microcrystalline cellulose as described in the prior art such as EP 122 036 results in a system that can undergo separation of particles on storage, shipment and administration. For example, when evaluated using an Andersen Impactor, the insulin was found largely in the smaller size fractions and the cellulose in the larger fractions. This could lead to non-uniformity of dosing and unpredictive absorption. Greater control over the deposition site in the nose can be achieved with smaller and more uniform particles.

Preferred aspects of the invention will now be illustrated by way of example and with reference to the accompanying drawings, in which:

Figure 1 shows the results of Example 3, illustrating intranasal administration of insulin at 2 IU/kg with 2 mg/kg of differently sized microspheres in sheep.

Figure 2 shows the results of Example 4, illustrating the clearance of intranasally-administered, radiolabelled, large and small microsphere formulations and a liquid formulation, in humans.

## EXAMPLE 1: Production of albumin microspheres

Albumin microspheres were produced by a modification of the method described by Ratcliffe *et al* (1984) *J. Pharm. Pharmacol.* **36**, 431-436, which is incorporated herein by reference. One ml of 5% human serum albumin or ovalbumin at pH 6.8 was added to 25 ml of olive oil or light mineral oil with or without 0.25 ml of Span 85. The mixture was stirred in a mix-cell for 10 min under turbulent flow conditions to form a w/o emulsion, using a mechanical stirrer (Heidolph) at 775 rpm (Tachometer DOT 1, Compact Instruments). Glutaraldehyde solution 25% (w/v) was added to 3.6% (v/v) of aqueous phase and the emulsion stirred for a further 30 min to denature and cross-link the albumin. The microspheres were collected by centrifugation at 2500 g for 20 min. The oil was then removed and the spheres washed with diethyl ether followed by ethanol. The microspheres were collected by decantation.

## EXAMPLE 2: Production of starch microspheres

5 g potato starch were dissolved in 95 ml of water at about 90°C. A second solution was prepared from 3 g of polyethylene glycol ($m_w$=6000) and 47 ml of water. This solution was heated to about 70°C, whereafter the warm starch solution was added while stirring, to form an emulsion. When the two-phase system had formed (with the starch solution as the inner phase) the mixture was allowed to cool to room temperature under continued stirring, whereupon the inner phase was converted to gel particles. The particles were filtered off at room temperature and stirred in 100 ml of ethanol, whereafter the particles were again filtered off and laid to dry in air.

The yield was 90%.

## EXAMPLE 3: Comparative Biological Data (sheep)

**Summary.** Insulin was administered nasally to sheep at 2 IU/kg as a lyophilised powder with either starch microspheres 45/25 (SMS 45/25) or smaller (<10 microns) starch microspheres BR 71B 03C (SSMS BR 71B) at 2 mg/kg. After an initial small rise in both groups, the plasma glucose concentrations were generally lower after SSMS BR 71B co-administration. The lowest concentrations reached after SSMS BR 71B and SMS 45/25 co-administration were 82.0% and 86.2% of control at 150 minutes after dosing.

### Materials and Methods

Semi-synthetic human Na-insulin (Nordisk, Gentofte, Batch No P389, 28 IU/mg) was used. The water content of the sample was determined by spectrophotometry, and the material was found to be 84.4% pure. Starch microspheres 45/25, Batch Number 49238) and smaller (<10 microns) starch microspheres BR 71B 03C (SSMS BR 71B, Batch Number 97327b), were supplied by Pharmacia.

Ultra pure water ("Elgastat UHP", Elga) was used throughout and all other reagents were at least of standard laboratory grade.

**Sheep.** Eight male cross-bred sheep weighing (± SEM) 29.0 ± 1.07 kg were used. The sheep were normally housed indoors, and remained inside for the duration of the study. Animals were not fasted prior to insulin administration. An in-dwelling Viggo secalon cannula of 1.2 mm id, fitted with a secalon universal flow-switch, was placed approximately 15 cm into one of the external jugular veins of each animal on the first day of the study and, whenever necessary, kept patent by flushing it with heparinised (25 IU/ml) 0.9% saline solution. This cannula was removed upon the completion of the study and the sheep were returned to their normal housing.

**Preparation of insulin formulations.** For the preparation of these lyophilised microsphere formulations, a solution of 50.8 mg insulin in 50 ml of water (1.016 mg/ml, 24 IU/ml) was prepared. The required quantity of each of the microspheres, SMS 45/25 or SSMS BR 71B (480 mg), was dispersed in 20 ml of insulin solution plus 12 ml of water (to keep the ratio of microspheres to solution at 15:1 [mg:ml]). The two resultant suspensions were stirred for one hour at room temperature and then freeze-dried to obtain the powder formulations (Formulations 1 and 2). The freeze-drying was performed on an Edwards Modulyo freeze-dryer fitted with a bell-jar assembly and operated at a pressure of 0.08 torr (10.7 $N/m^2$), a condenser temperature of -53°C and a product shelf temperature of approximately 20°C. The freeze-drying process was allowed to proceed for 24 hours, after which the final product was loaded into the administration devices and then stored with dessicant at 4°C for 16 hours prior to administration to the sheep.

**Administration of insulin formulations and blood sampling.** The sheep were divided into two groups of four animals each. Group 1: Four animals received 2.0 IU/kg insulin together with 2.0 mg/kg SMS 45/25 microspheres (Formulation 1) intranasally in the form of a lyophilised powder. A sheep of 30 kg thus received 60 IU of insulin together with 60 mg SMS 45/25 microspheres. Group 2: Four animals received 2.0 IU/kg insulin together with 2.0 mg/kg SSMS BR 71B microspheres (Formulation 2) intranasally in the form of a lyophilised powder. A sheep of 30 kg thus received 60 IU of insulin together with 60 mg SSMS BR 71B microspheres.

The sheep were sedated with an iv dose of ketamine hydrochloride (Ketalar[R], 100 mg/ml injection) at 2.25 mg/kg and this anaesthesia lasted for about 3 minutes. This treatment acted as an animal restraint, and also as a counter-measure against the animal sneezing during administration. For intranasal administration a Leymed red rubber Magill's tube oral of 6.5 mm was loaded with the powder formulation and then inserted into the nostril of the sheep to a preset depth of 6 cm before blowing the powder into the nasal cavity. Blood samples of 6.0 ml were collected onto crushed ice from the cannulated jugular vein of the sheep at 15 and 5 minutes prior to the insulin administration and at 5, 10, 15, 20, 30, 40, 50, 60, 75, 90, 120, 150, 180 and 240 minutes post-administration. Each blood sample was divided into two parts. For insulin analysis, the blood collected (4.0 ml) was mixed gently in 4 ml heparinised tubes (Lithium Heparin, 60 IU, Sarstedt, Leicester, UK). For glucose analysis, the blood collected (2.0 ml) was mixed gently in 2 ml sodium fluoride tubes (2.0 mg fluoride and 30 IU heparin, Sarstedt, Leicester, UK). The plasma was separated by centrifugation at 4°C and 3200 rpm, and then stored at -20°C awaiting insulin and glucose analysis within our Analytical Section.

**Analysis.** Plasma glucose concentrations were analysed by the glucose oxidase method using a Yellow Springs 23 AM glucose analyser (Yellow Springs, Ohio, USA). Plasma insulin was not measured at this stage.

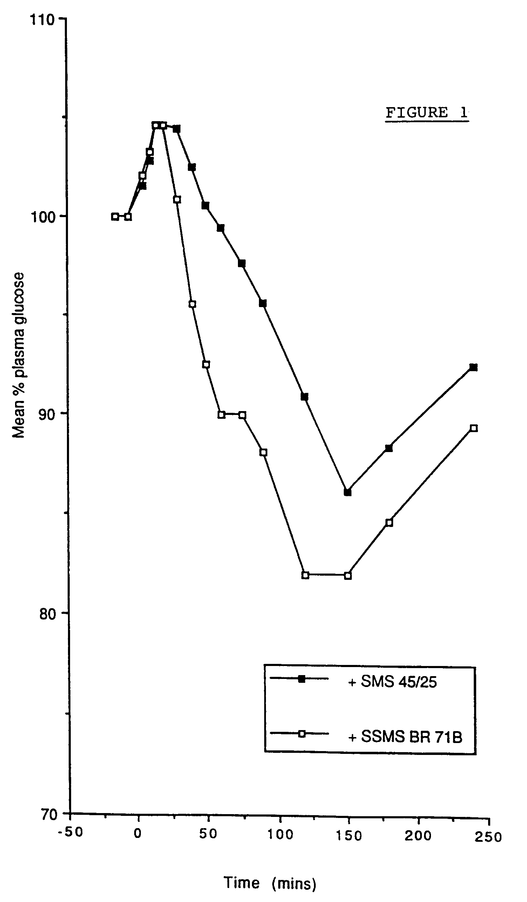

**Results and discussion.** The mean changes in plasma glucose following the co-administration of insulin with SMS 45/25 or SSMS BR 71B are plotted on the same axes in Figure 1.

Both SMS 45/25 and SSMS BR 71B groups showed an initial rise in plasma glucose concentrations up to approximately 105.0% of the controls at approximately 20-30 minutes after dosing. Thereafter, concentrations of glucose steadily fell to a low point at approximately 150 minutes after dosing. At this low point the change in glucose concentration was greatest after co-administration of SSMS BR 71B (82.0%) than after co-administration of SMS 45/25 (86.2%). Indeed, after co-administration of SSMS BR 71B the glucose concentrations were generally lower than those after SMS 45/25 co-administration.

The area under the curve (AUC) is a particularly important measure of the effectiveness of the enhancer system: Group 1 gave a mean AUC of 1704% per minute, whereas Group 2 gave a mean AUC of 2766% per minute. This is a 62% increase in AUC, and was accompanied by a 25% increase in the blood concentration of insulin.

## EXAMPLE 4: Comparative Biological Data (human)

Small starch microspheres were labelled with Tc99m using the stannous chloride technique and freeze dried. Doses of 30 mg were filled into hard gelatin capsules for the application. A group of healthy male and female volunteers (n=3) were each given the microsphere preparation using a Lomudal nasal insufflator. The total content of the capsules was applied during inhalation to the mucosal surface of either the right or the left nostril. The deposition and the subsequent clearance of the formulation were followed by gamma scintigraphy while the volunteers were positioned in an upright position with the nose in a fixed position close to the collimator surface of the camera, using a specially designed template. Static views were recorded at times 0, 10, 30, 45, 90, 120, 180 and 240 min after administration. Regions of interest were drawn around the initial site of deposition and the total nasal cavity. The counts from each region were corrected for background counts and radioactive decay and expressed as a portion of the registered activity in the initial deposition site.

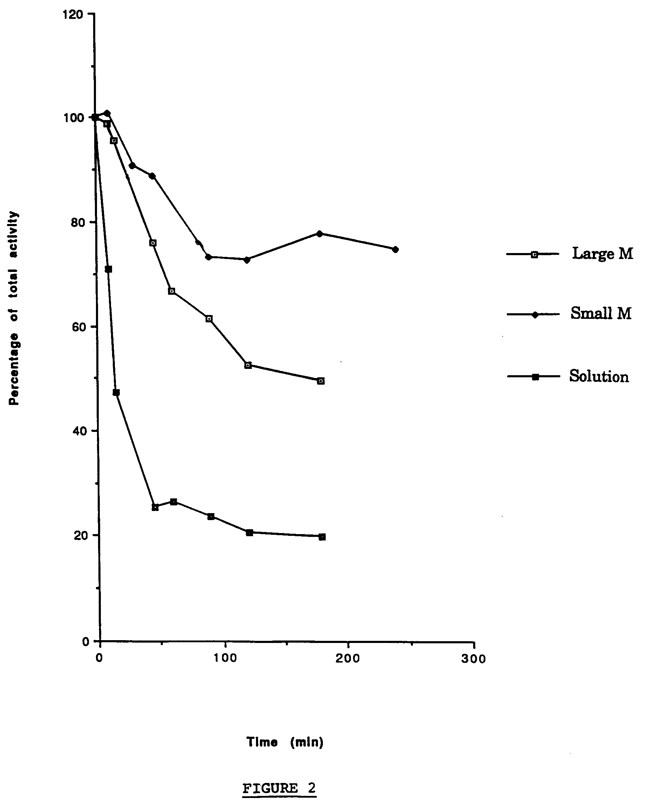

Fig 2 shows a comparison between the clearance of small starch microspheres and large microspheres and a simple control solution. It can be seen that the half time of clearance for the small microspheres has not been reached within the time of the study, whereas the half time of clearance for the large microspheres is 180 min as compared to 15 min for the solution formulation.

Hence the use of the small microspheres (1-10 µm) as compared to the large microspheres (40 µm) gives a significantly longer residence time in the nasal cavity.

## Claims

### Claims for the following Contracting States : AT, BE, CH, DE, DK, FR, GB, IT, LU, NL, SE

1. A drug delivery composition for intranasal delivery comprising a plurality of bioadhesive microspheres and active drug associated with or forming at least part of each microsphere, at least 90 wt % of the microspheres having a diameter of 0.1 µm or more but less than 10 µm characterised in that the drug is one for which systemic delivery is desired.

2. A drug delivery composition according to Claim 1 wherein the microspheres are prepared from a material that will gel in contact with the mucosal surface.

3. A drug delivery composition according to Claim 1 or Claim 2 wherein the microspheres comprise starch, starch derivatives, gelatin, albumin, collagen, dextran or dextran derivatives.

4. A drug delivery composition according to Claim 3 wherein the microspheres are starch microspheres.

5. A drug delivery composition according to Claim 3 or Claim 4 wherein the microsphere material is at least partially cross-linked.

6. A drug delivery composition according to Claim 1 or Claim 2 wherein the microspheres are formed from the active drug itself.

7. A drug delivery composition according to any one of the preceding claims additionally comprising an absorption enhancer.

8. A drug delivery composition according to Claim 7 wherein the absorption enhancer is a surfactant, a lysophosphatidylcholine or a lysophosphatidylglycerol.

9. A drug delivery composition according to any one of the preceding claims wherein the drug is a biologically active peptide.

10. A drug delivery composition according to Claim 9 wherein the peptide is insulin or calcitonin.

11. A kit comprising a drug delivery composition according to any one of the preceding claims and means to deliver the composition to the nasal mucosa in a gas stream.

12. A kit according to Claim 11 wherein the means is such that, in use, the product of the flow rate and the square of the microsphere aerodynamic diameter is greater than 2000 $\mu m^2$.litres/min.

**Claims for the following Contracting States : ES, GR**

1. A method of mailing a drug delivery composition for intranasal delivery comprising preparing a plurality of bioadhesive microspheres, at least 90 wt % of the microspheres having a diameter of 0.1 $\mu m$ or more but less than 10 $\mu m$ and incorporating a drug for which systemic delivery is desired into the microspheres during their formulation or sorbing the drug into or onto the microspheres after their preparation.

2. A method according to Claim 1 wherein the microspheres are prepared from a material that will gel in contact with the mucosal surface.

3. A method according to any one of Claims 1 or 2 wherein the microspheres comprise starch, starch derivatives, gelatin, albumin, collagen, dextran or dextran derivatives.

4. A method according to Claim 3 wherein the microspheres are starch microspheres.

5. A method according to Claim 3 or Claim 4 wherein the microsphere material is at least partially cross-linked.

6. A method according to Claim 1 or 2, wherein the microspheres are formed from the active drug itself.

7. A method according to any one of the preceding claims additionally comprising admixing an absorption enhancer, with the microspheres and active drug.

8. A method according to Claim 7 wherein the absorption enhancer is a surfactant, a lysophosphatidylcholine or a lysophosphatidylglycerol.

9. A method according to any one of the preceding claims wherein the drug is a biologically active peptide.

10. A method according to Claim 9 wherein the peptide is insulin or calcitonin.

11. A kit comprising a drug delivery composition according to any one of the preceding claims and means to deliver the composition to the nasal mucosa in a gas stream.

12. A kit according to Claim 11 wherein the means is such that, in use, the product of the flow rate and the square of the microsphere aerodynamic diameter is greater than 2000 $\mu m^2$.litres/min.

**Patentansprüche**

**Patentansprüche für folgende Vertragsstaaten : AT, BE, CH, DE, DK, FR, GB, IT, LU, NL, SE**

1. Arzneimittelabgabezusammensetzung zur intranasalen Abgabe, umfassend eine Mehrzahl von biohaftenden Mikrokügelchen und ein mit jedem Mikrokügelchen vereinigtes oder zumindest einen Teil eines jeden Mikrokügelchens bildendes aktives Arzneimittel, wobei mindestens 90 Gew.-% der Mikrokügelchen einen Durchmesser von 0,1 $\mu m$ oder mehr, jedoch weniger als 10 $\mu m$ aufweisen, dadurch gekennzeichnet, daß es sich bei dem Arzneimittel um ein zur systemischen Abgabe vorgesehenes Arzneimittel handelt.

2. Arzneimittelabgabezusammensetzung nach Anspruch 1, wobei die Mikrokügelchen aus einem bei Kontakt mit der Schleimhautoberfläche gelierenden Material hergestellt sind.

3. Arzneimittelabgabezusammensetzung nach einem der Ansprüche 1 oder 2, wobei die Mikrokügelchen Stärke, Stärkederivate, Gelatine, Albumin, Collagen, Dextran oder Dextranderivate umfassen.

4. Arzneimittelabgabezusammensetzung nach Anspruch 3, wobei die Mikrokügelchen aus Stärkemikrokügelchen bestehen.

5. Arzneimittelabgabezusammensetzung nach Ansprüchen 3 oder 4, wobei das Mikrokügelchenmaterial zumindest teilweise vernetzt ist.

6. Arzneimittelabgabezusammensetzung nach Ansprüchen 1 oder 2, wobei die Mikrokügelchen aus dem aktiven Arzneimittel selbst gebildet sind.

7. Arzneimittelabgabezusammensetzung nach einem der vorhergehenden Ansprüche, welche zusätzlich einen Absorptionsverstärker umfaßt.

8. Arzneimittelabgabezusammensetzung nach Anspruch 7, wobei der Absorptionsverstärker aus einem oberflächenaktiven Mittel bzw. Netzmittel, einem Lysophosphatidylcholin oder einem Lysophosphatidylglycerin besteht.

9. Arzneimittelabgabezusammensetzung nach einem der vorhergehenden Ansprüche, wobei das Arzneimittel aus einem biologisch aktiven Peptid besteht.

10. Arzneimittelabgabezusammensetzung nach Anspruch 9, wobei das Peptid aus Insulin oder Calcitonin besteht.

11. Besteck, umfassend eine Arzneimittelabgabezusammensetung nach einem der vorhergehenden Ansprüche und eine Vorrichtung zur Abgabe der Zusammensetzung an die Nasenschleimhaut in einem Gasstrom.

12. Besteck nach Anspruch 11, wobei die Vorrichtung derart ausgestaltet ist, daß - in Gebrauch - das Produkt aus der Strömungsgeschwindigkeit und dem Quadrat des aerodynamischen Mikrokügelchendurchmessers größer als 2000 $\mu m^2 \cdot$ l/min ist.

**Patentansprüche für folgende Vertragsstaaten : ES, GR**

1. Verfahren zur Herstellung einer Arzneimittelabgabezusammensetzung zur intranasalen Abgabe durch Herstellen einer Mehrzahl von biohaftenden Mikrokügelchen, von denen mindestens 90 Gew.-% einen Durchmesser von 0,1 $\mu m$ oder mehr, jedoch weniger als 10 $\mu m$, aufweisen und Einarbeiten eines zur systemischen Abgabe vorgesehenen Arzneimittels in die Mikrokügelchen während ihrer Bildung oder Sorbieren des Arzneimittels in oder auf die Mikrokügelchen nach ihrer Herstellung.

2. Verfahren nach Anspruch 1, wobei die Mikrokügelchen aus einem bei Kontakt mit der Schleimhautoberfläche gelierenden Material hergestellt sind.

3. Verfahren nach einem der Ansprüche 1 oder 2, wobei die Mikrokügelchen Stärke, Stärkederivate, Gelatine, Albumin, Collagen, Dextran oder Dextranderivate umfassen.

4. Verfahren nach Anspruch 3, wobei die Mikrokügelchen aus Stärkemikrokügelchen bestehen.

5. Verfahren nach Ansprüchen 3 oder 4, wobei das Mikrokügelchenmaterial zumindest teilweise vernetzt ist.

6. Verfahren nach Ansprüchen 1 oder 2, wobei die Mikrokügelchen aus dem aktiven Arzneimittel selbst gebildet sind.

7. Verfahren nach einem der vorhergehenden Ansprüche, bei welchem zusätzlich ein Absorptionsverstärker mit den Mikrokügelchen und dem aktiven Arzneimittel gemischt wird.

8. Verfahren nach Anspruch 7, wobei der Absorptionsverstärker aus einem oberflächenaktiven Mittel bzw. Netzmittel, einem Lysophosphatidylcholin oder einem Lysophosphatidylglycerin besteht.

9. Verfahren nach einem der vorhergehenden Ansprüche, wobei das Arzneimittel aus einem biologisch aktiven Peptid besteht.

10. Verfahren nach Anspruch 9, wobei das Peptid aus Insulin oder Calcitonin besteht.

11. Besteck, umfassend eine Arzneimittelabgabezusammensetung nach einem der vorhergehenden Ansprüche und eine Vorrichtung zur Abgabe der Zusammensetzung an die Nasenschleimhaut in einem Gasstrom.

12. Besteck nach Anspruch 11, wobei die Vorrichtung derart ausgestaltet ist, daß - in Gebrauch - das Produkt aus der Strömungsgeschwindigkeit und dem Quadrat des aerodynamischen Mikrokügelchendurchmessers größer als 2000 $\mu m^2 \cdot$ l/min ist.

**Revendications**

**Revendications pour les Etats contractants suivants : AT, BE, CH, DE, DK, FR, GB, IT, LU, NL, SE**

1. Composition pour la délivrance de médicament, pour délivrance intranasale, comprenant une pluralité de microsphères bioadhésives et un médicament actif associé avec chaque microsphère ou formant au moins une partie de celle-ci, au moins 90 % en poids des microsphères ayant un diamètre de 0,1 $\mu m$ ou plus mais inférieur à 10 $\mu m$, caractérisée en ce que le médicament est un médicament pour lequel une délivrance systémique est souhaitée.

2. Composition pour la délivrance de médicament selon la revendication 1, dans laquelle les microsphères sont préparées à partir d'une matière qui va se gélifier au contact avec la surface muqueuse.

3. Composition pour la délivrance de médicament selon l'une quelconque des revendications 1 ou 2, dans laquelle les microsphères comprennent de l'amidon, des dérivés de l'amidon, de la gélatine, de l'albumine, du collagène, du dextrane ou des dérivés de dextrane.

4. Composition pour la délivrance de médicament selon la revendication 3, dans laquelle les microsphères sont des microsphères d'amidon.

5. Composition pour la délivrance de médicament selon la revendication 3 ou la revendication 4, dans laquelle la matière des microsphères est au moins partiellement réticulée.

6. Composition pour la délivrance de médicament selon la revendication 1 ou 2, dans laquelle les microsphères sont formées à partir du médicament actif lui- même.

7. Composition pour la délivrance de médicament selon l'une quelconque des revendications précédentes, comprenant en plus un activateur d'absorption.

8. Composition pour la délivrance de médicament selon la revendication 7, dans laquelle l'activateur d'absorption est un agent tensioactif, une lyosphosphatidylcholine ou un lysophosphatidylglycérol.

9. Composition pour la délivrance de médicament selon l'une quelconque des revendications précédentes, dans laquelle le médicament est un peptide biologiquement actif.

10. Composition pour la délivrance de médicament selon la revendication 9, dans laquelle le peptide est l'insuline ou la calcitonine.

11. Nécessaire comprenant une composition pour la délivrance de médicament selon l'une quelconque des revendications précédentes et des moyens pour délivrer la composition à la muqueuse nasale dans un courant de gaz.

12. Nécessaire selon la revendication 11, dans lequel les moyens sont tels que, lors de l'utilisation, le produit du débit et du carré du diamètre aérodynamique des microsphères est supérieur à 2 000 $\mu m^2$.litres/min.

**Revendications pour les Etats contractants suivants : ES, GR**

1. Procéde de préparation d'une composition pour la délivrance de médicament, pour délivrance intranasale, comprenant la préparation d'une pluralité de microsphères bioadhésives, au moins 90 % en poids des microsphères ayant un diamètre de 0,1 $\mu m$ ou plus mais inférieur à 10 $\mu m$, et l'incorporation dans les microsphères, pendant leur formulation, d'un médicament pour lequel une délivrance systémique est souhaitée, ou la sorption du médicament dans ou sur les microsphères après leur préparation.

2. Procédé selon la revendication 1, dans lequel les microsphères sont préparées à partir d'une matière qui va se gélifier au contact avec la surface muqueuse.

3. Procédé selon l'une quelconque des revendications 1 ou 2, dans lequel les microsphères comprennent de l'amidon, des dérivés de l'amidon, de la gélatine, de l'albumine, du collagène, du dextrane ou des dérivés de dextrane.

4. Procédé selon la revendication 3, dans lequel les microsphères sont des microsphères d'amidon.

5. Procédé selon la revendication 3 ou la revendication 4, dans lequel la matière des microsphères est au moins partiellement réticulée.

6. Procédé selon la revendication 1 ou 2, dans lequel les microsphères sont formées à partir du médicament actif lui-même.

7. Procédé selon l'une quelconque des revendications précédentes, comprenant en plus le mélange d'un activateur d'absorption, avec les microsphères et le médicament actif.

8. Procédé selon la revendication 7, dans lequel l'activateur d'absorption est un agent tensioactif, une lyosphosphatidylcholine ou un lysophosphatidylglycérol.

9. Procédé selon l'une quelconque des revendications précédentes, dans lequel le médicament est un peptide biologiquement actif.

10. Procédé selon la revendication 9, dans lequel le peptide est l'insuline ou la calcitonine.

11. Nécessaire comprenant une composition pour la délivrance de médicament selon l'une quelconque des revendications précédentes et des moyens pour délivrer la composition à la muqueuse nasale dans un courant de gaz.

12. Nécessaire selon la revendication 11, dans lequel les moyens sont tels que, lors de l'utilisation, le produit du débit et du carré du diamètre aérodynamique des microsphères est supérieur à 2 000 $\mu m^2$.litres/min.

FIGURE 1

**Time (min)**

FIGURE 2